(19) Europäisches Patentamt
European Patent Office
Office européen des brevets

(11) EP 4 209 209 A1

(12) EUROPEAN PATENT APPLICATION
published in accordance with Art. 153(4) EPC

(43) Date of publication:
**12.07.2023 Bulletin 2023/28**

(21) Application number: **21847374.2**

(22) Date of filing: **22.07.2021**

(51) International Patent Classification (IPC):
***A61K 31/137*** *(2006.01)* ***A61K 45/06*** *(2006.01)*
***A61P 31/14*** *(2006.01)*

(86) International application number:
**PCT/CN2021/107739**

(87) International publication number:
**WO 2022/017431 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.07.2020 CN 202010722678**

(71) Applicant: **Academy of Military Medical Sciences**
**Beijing 100850 (CN)**

(72) Inventors:
- **CAO, Ruiyuan**
  **Beijing 100850 (CN)**
- **WANG, Manli**
  **Beijing 100850 (CN)**
- **LI, Wei**
  **Beijing 100850 (CN)**
- **ZHAO, Lei**
  **Beijing 100850 (CN)**
- **YANG, Jingjing**
  **Beijing 100850 (CN)**
- **LI, Yuexiang**
  **Beijing 100850 (CN)**
- **FAN, Shiyong**
  **Beijing 100850 (CN)**
- **ZHOU, Xinbo**
  **Beijing 100850 (CN)**
- **XIAO, Dian**
  **Beijing 100850 (CN)**
- **HU, Zhihong**
  **Beijing 100850 (CN)**
- **LI, Song**
  **Beijing 100850 (CN)**
- **ZHONG, Wu**
  **Beijing 100850 (CN)**

(74) Representative: **Inspicos P/S**
**Agern Allé 24**
**2970 Hørsholm (DK)**

Remarks:
The complete document including Reference Table(s) and the Sequence Listing(s) can be downloaded from the EPO website

# (54) APPLICATION OF BENFLUMETOL AND DERIVATIVES THEREOF IN TREATMENT OF CORONAVIRUS INFECTION

(57) The present invention relates to an application of benflumetol and derivatives thereof in treatment of coronavirus infection, and specifically provides uses of a compound represented by formula A, and a stereoisomer, a pharmaceutically acceptable salt, a solvate or a hydrate thereof in preparation of drugs. The drugs are used for treating diseases or infection caused by coronavirus (preferably SARS-CoV-2).

EP 4 209 209 A1

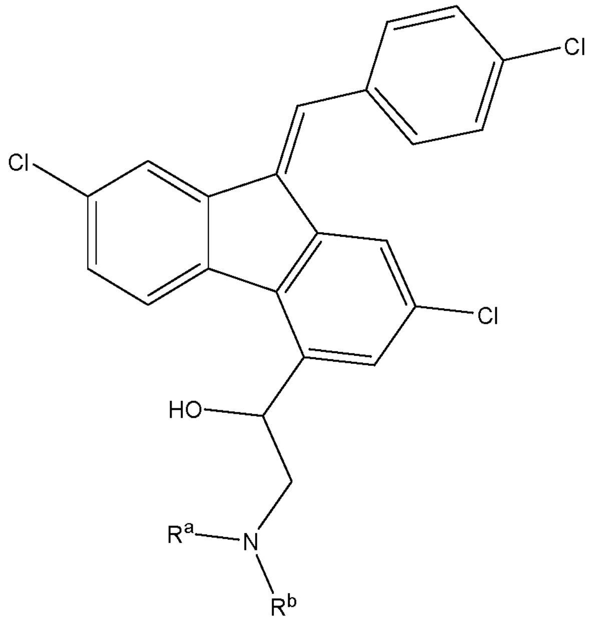

A

## Description

### Technical Field

The present invention relates to the field of biomedicine, in particular to the application of benflumetol and derivatives thereof in the treatment of coronavirus infection.

### Background Art

Lumefantrine/benflumetol is the main active ingredient of the marketed drug compound artemether-benflumetol tablets (trade name: Coartem/Riamet), which has good antimalarial activity. Compound benflumetol has been approved for the treatment of patients weighing more than Skg (including Skg) with acute simple infection caused by *Plasmodium falciparum.* At the same time, compound benflumetol also has an ideal therapeutic effect on patients infected with *falciparum malaria* in chloroquine-resistant areas. Compound artemether-benflumetol tablets (Artemether-Lumefantrine) contain 20mg of artemether and 120mg of benflumetol. The two are used together as the main active ingredients, which helps to complement each other's antimalarial effects, and show significant therapeutic effects on *Plasmodium falciparum* and diseases thereof. In addition to the use in malaria treatment, benflumetol has strong antitumor activity, which can effectively inhibit the growth of prototype and drug-resistant glioblastoma in in vitro and in vivo models. In addition, the results of clinical trials show that compound benflumetol may have a certain inhibitory effect on human cytomegalovirus. At present, compound artemether-benflumetol tablets have been approved for marketing in China, the United States, Switzerland and other countries around the world.

The 2019 novel coronavirus (2019-nCoV) is a new strain of coronavirus that has never been found in humans before. On February 11, 2020, the International Committee on Taxonomy of Viruses (ICTV) announced that the official classification of the 2019 novel coronavirus (2019-nCoV) is severe acute respiratory syndrome coronavirus 2 (SARS-CoV-2). On the same day, the World Health Organization (WHO) announced that the official name of the disease caused by the virus is COVID-19. The symptoms of SARS-CoV-2 infection are mainly pneumonia, which can be divided into simple infection, mild pneumonia, severe pneumonia, acute respiratory distress syndrome, sepsis, and septic shock according to the severity of the disease. Patients with simple infection may have nonspecific symptoms such as fever, cough, sore throat, nasal congestion, fatigue, headache, muscle pain or discomfort, and elderly and immunosuppressed individuals may experience atypical symptoms. Patients with mild pneumonia mainly have symptoms of cough, dyspnea and tachypnea. Severe pneumonia can be seen in adolescents, adults, or children. And the main symptoms of severe pneumonia are increased respiratory rate, severe respiratory failure or dyspnea, central cyanosis, lethargy, unconsciousness or convulsions, and gasp, etc. The lung images of acute respiratory distress syndrome are bilateral ground-glass opacities, which cannot be completely explained by effusion, lobar exudation, atelectasis, or pulmonary mass. And the main symptom of acute respiratory distress syndrome is pulmonary edema. Patients with sepsis often have fatal organ dysfunction, and patients with septic shock are the most critical patients with a high probability of death. At present, for the new coronavirus infection, supportive treatment is mainly used in clinic, and no specific antiviral drug is available.

### Contents of the Invention

The purpose of the present invention is to find a drug with antiviral activity against coronavirus, especially SARS-CoV-2, which can be used for a related disease caused by the infection thereof, for example simple infection such as fever, cough and sore throat, pneumonia, acute or severe acute respiratory infection, hypoxic respiratory failure and acute respiratory distress syndrome, sepsis and septic shock, etc. Through creative research, the present invention finds that the compound represented by Formula A (benflumetol and derivative thereof) has the function of inhibiting the replication of SARS-CoV-2, and has a good potential therapeutic effect in the treatment of a disease caused by SARS-CoV-2.

To this end, in the first aspect of the present invention, the present invention provides use of the compound represented by Formula A, or a stereoisomer, a pharmaceutically acceptable salt, a solvate or a hydrate thereof in the manufacture of a medicament for the treatment of a disease or infection caused by a coronavirus (preferably SARS-CoV-2);

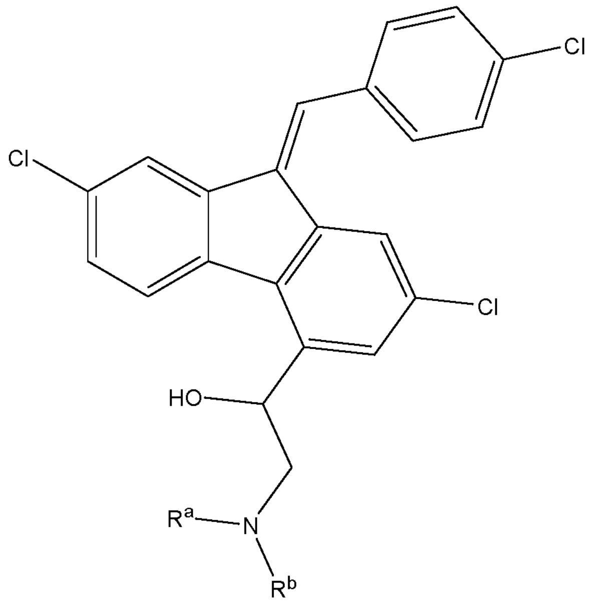

Formula A

wherein:
$R^a$ and $R^b$ are each independently selected from the group consisting of H, $C_1$-$C_6$ alkyl.

The inventors found that the compound represented by Formula A can inhibit viral replication on cells and reduce the viral nucleic acid load in cell culture.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea, and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, $R^a$, $R^b$ are each independently selected from the group consisting of n-propyl, isopropyl, n-butyl, isobutyl, tert-butyl, sec-butyl.

In some embodiments, $R^a$, $R^b$ are each independently selected from the group consisting of n-butyl, isobutyl, tert-butyl, sec-butyl.

In some embodiments, the structure of the compound of Formula A is represented by Formula I,

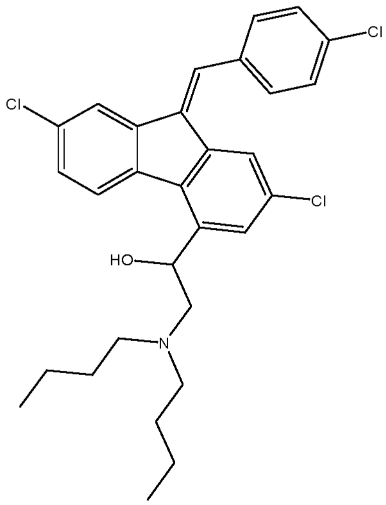

Formula I.

In the second aspect of the present invention, the present invention provides use of a pharmaceutical composition in the manufacture of a medicament for treating a disease or infection caused by a coronavirus (preferably SARS-CoV-2), wherein the pharmaceutical composition comprises the compound represented by Formula A, or a stereoisomer, a pharmaceutically acceptable salt, a solvate or a hydrate thereof, and the compound represented by Formula A is as described above.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea, and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, the pharmaceutical composition further comprises one or more selected from the group consisting of arteether, artemether, artemisone, dihydroartemisinin, artesunate, arteannuin B, artemisinic acid, and artemisinin. In some preferred embodiments, the pharmaceutical composition further comprises artemether, for example, it can be a compound artemether-benflumetol tablet.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the third aspect of the present invention, the present invention provides the compound represented by Formula A, or a stereoisomer, a pharmaceutically acceptable salt, a solvate or a hydrate thereof, for use in the treatment of a disease or infection caused by a coronavirus (preferably SARS-CoV-2);

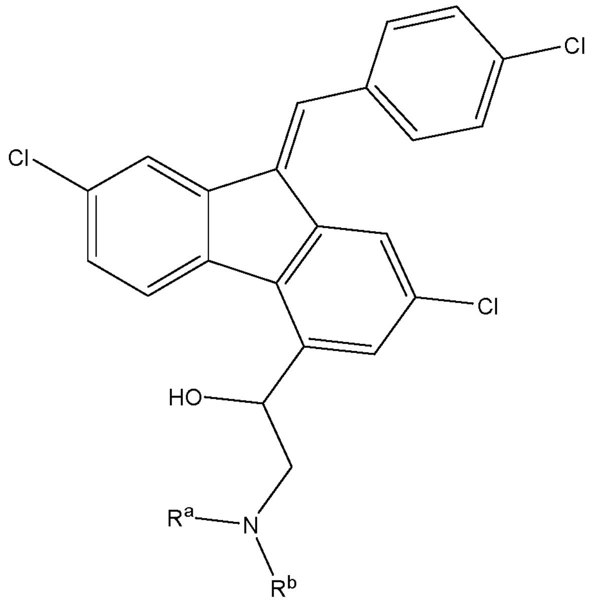

Formula A

wherein:

$R^a$ and $R^b$ are each independently selected from the group consisting of H, $C_1$-$C_6$ alkyl.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea, and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, $R^a$, $R^b$ are each independently selected from the group consisting of n-propyl, isopropyl, n-butyl, isobutyl, tert-butyl, sec-butyl.

In some embodiments, $R^a$, $R^b$ are each independently selected from the group consisting of n-butyl, isobutyl, tert-butyl, sec-butyl.

In some embodiments, the structure of the compound of Formula A is represented by Formula I,

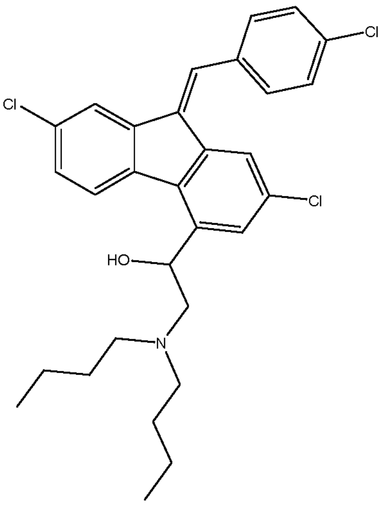

Formula I.

In the fourth aspect of the present invention, the present invention provides a pharmaceutical composition, for use in treating a disease or infection caused by a coronavirus (preferably SARS-CoV-2), wherein the pharmaceutical composition comprises the compound represented by Formula A, or a stereoisomer, a pharmaceutically acceptable salt, a solvate or a hydrate thereof, and the compound represented by Formula A is as described above.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea, and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, the pharmaceutical composition further comprises one or more selected from the group consisting of arteether, artemether, artemisone, dihydroartemisinin, artesunate, arteannuin B, artemisinic acid, and artemisinin. In some preferred embodiments, the pharmaceutical composition further comprises artemether, for example, it can be a compound artemether-benflumetol tablet.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the fifth aspect of the present invention, the present invention provides a method for treating a disease, which comprises administering to a subject in need thereof a therapeutically effective amount of the compound represented by Formula A, or a stereoisomer, a pharmaceutically acceptable salt, a solvate or a hydrate thereof, the disease being a disease or infection caused by a coronavirus, preferably SARS-CoV-2,

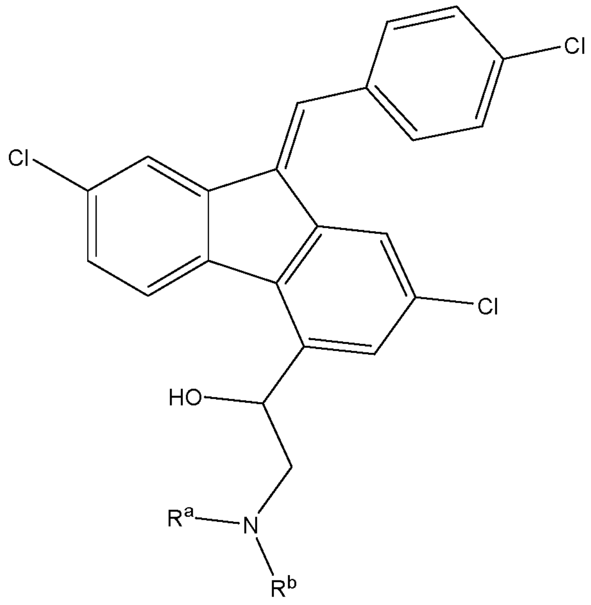

Formula A

wherein:
$R^a$ and $R^b$ are each independently selected from the group consisting of H, $C_1$-$C_6$ alkyl.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea, and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, $R^a$, $R^b$ are each independently selected from the group consisting of n-propyl, isopropyl, n-butyl, isobutyl, tert-butyl, sec-butyl.

In some embodiments, $R^a$, $R^b$ are each independently selected from the group consisting of n-butyl, isobutyl, tert-butyl, sec-butyl.

In some embodiments, the structure of the compound of Formula A is represented by Formula I,

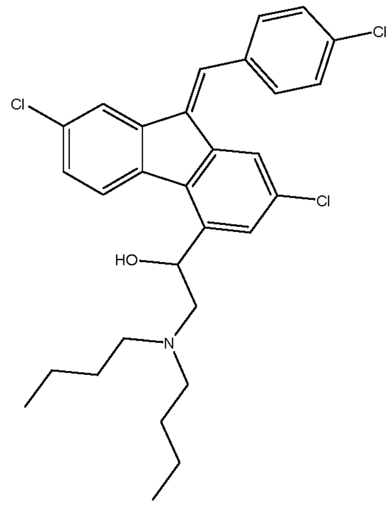

Formula I.

In the sixth aspect of the present invention, the present invention provides a method for treating a disease, comprising administering to a subject in need thereof, a therapeutically effective amount of a pharmaceutical composition, wherein the disease is a disease or infection caused by a coronavirus (preferably SARS-CoV-2), the pharmaceutical composition comprises the compound represented by Formula A, or a stereoisomer, a pharmaceutically acceptable salt, a solvate or a hydrate thereof, and the compound represented by Formula A is as described above.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea, and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, the pharmaceutical composition further comprises one or more selected from the group consisting of arteether, artemether, artemisone, dihydroartemisinin, artesunate, arteannuin B, artemisinic acid, and artemisinin. In some preferred embodiments, the pharmaceutical composition further comprises artemether, for example, it can be a compound artemether-benflumetol tablet.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the seventh aspect of the present invention, the present invention provides use of the compound represented by Formula A, or a stereoisomer, a pharmaceutically acceptable salt, a solvate or a hydrate thereof in the manufacture of a medicament as an inhibitor of a coronavirus (preferably SARS-CoV-2),

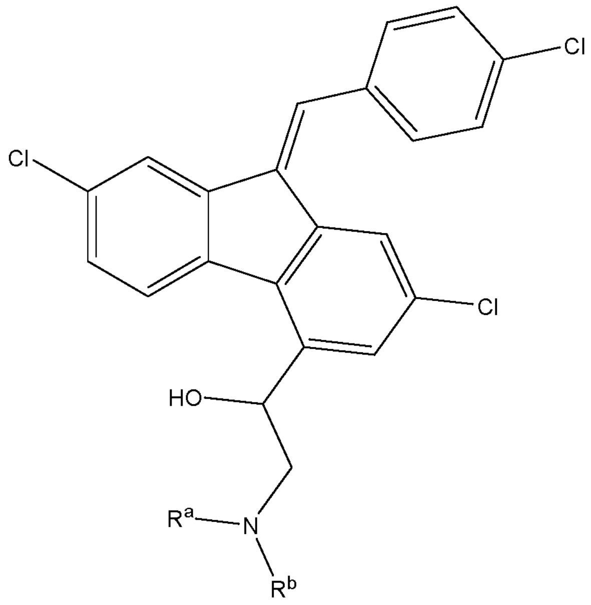

Formula A

wherein:

$R^a$ and $R^b$ are each independently selected from the group consisting of H, $C_1$-$C_6$ alkyl.

In some embodiments, $R^a$, $R^b$ are each independently selected from the group consisting of n-propyl, isopropyl, n-butyl, isobutyl, tert-butyl, sec-butyl.

In some embodiments, $R^a$, $R^b$ are each independently selected from the group consisting of n-butyl, isobutyl, tert-butyl, sec-butyl.

In some embodiments, the structure of the compound of Formula A is represented by Formula I,

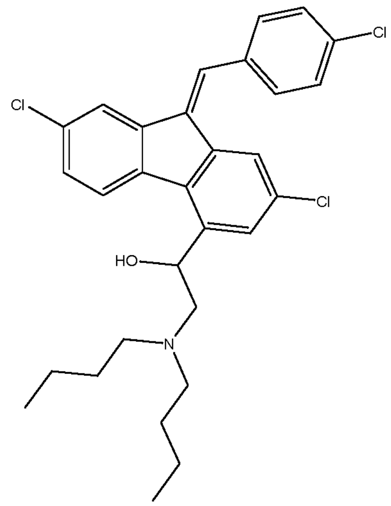

Formula I.

In the eighth aspect of the present invention, the present invention provides use of a pharmaceutical composition in the manufacture of a medicament as an inhibitor of a coronavirus (preferably SARS-CoV-2), wherein the pharmaceutical composition comprises the compound represented by Formula A, or a stereoisomer, a pharmaceutically acceptable salt, a solvate or a hydrate thereof, and the compound represented by Formula A is as described above.

In some embodiments, the pharmaceutical composition further comprises one or more selected from the group consisting of arteether, artemether, artemisone, dihydroartemisinin, artesunate, arteannuin B, artemisinic acid, and artemisinin. In some preferred embodiments, the pharmaceutical composition further comprises artemether, for example,

it can be a compound artemether-benflumetol tablet.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the ninth aspect of the present invention, the present invention provides the compound represented by Formula A, or a stereoisomer, a pharmaceutically acceptable salt, a solvate or a hydrate thereof, which is an inhibitor of a coronavirus (preferably SARS-CoV-2),

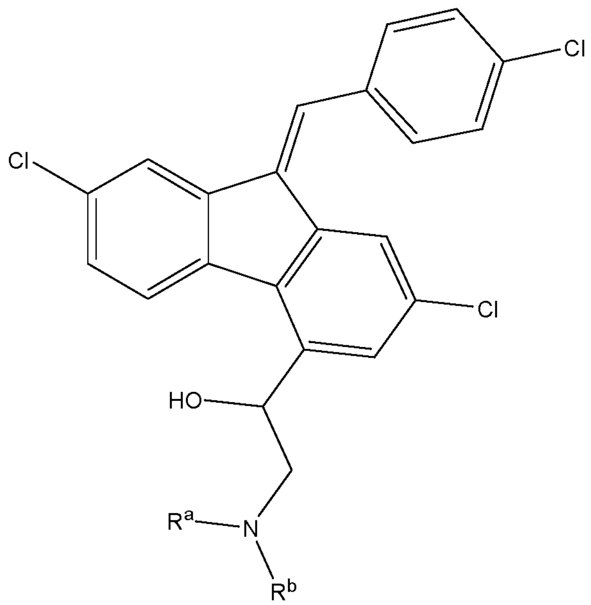

Formula A

wherein:

$R^a$ and $R^b$ are each independently selected from the group consisting of H, $C_1$-$C_6$ alkyl.

In some embodiments, $R^a$, $R^b$ are each independently selected from the group consisting of n-propyl, isopropyl, n-butyl, isobutyl, tert-butyl, sec-butyl.

In some embodiments, $R^a$, $R^b$ are each independently selected from the group consisting of n-butyl, isobutyl, tert-butyl, sec-butyl.

In some embodiments, the structure of the compound of Formula A is represented by Formula I,

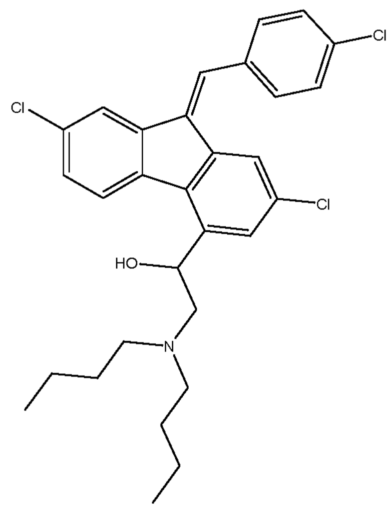

Formula I.

In the tenth aspect of the present invention, the present invention provides a pharmaceutical composition, which is an inhibitor of a coronavirus (preferably SARS-CoV-2), wherein the pharmaceutical composition comprises the compound represented by Formula A, or a stereoisomer, a pharmaceutically acceptable salt, a solvate or a hydrate thereof, and the compound represented by Formula A is as described above.

In some embodiments, the pharmaceutical composition further comprises one or more selected from the group consisting of arteether, artemether, artemisone, dihydroartemisinin, artesunate, arteannuin B, artemisinic acid, and artemisinin. In some preferred embodiments, the pharmaceutical composition further comprises artemether, for example, it can be a compound artemether-benflumetol tablet.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the eleventh aspect of the present invention, the present invention provides use of the compound represented by Formula A, or a stereoisomer, a pharmaceutically acceptable salt, a solvate or a hydrate thereof in the manufacture of a medicament for inhibiting the replication or proliferation of a coronavirus (preferably SARS-CoV-2) in a cell (e.g., mammalian cell);

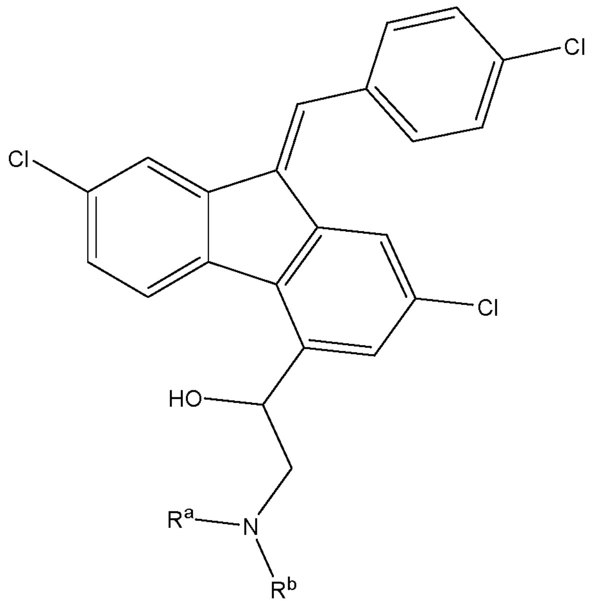

Formula A

wherein:

$R^a$ and $R^b$ are each independently selected from the group consisting of H, $C_1$-$C_6$ alkyl.

In some embodiments, $R^a$, $R^b$ are each independently selected from the group consisting of n-propyl, isopropyl, n-butyl, isobutyl, tert-butyl, sec-butyl.

In some embodiments, $R^a$, $R^b$ are each independently selected from the group consisting of n-butyl, isobutyl, tert-butyl, sec-butyl.

In some embodiments, the structure of the compound of Formula A is represented by Formula I,

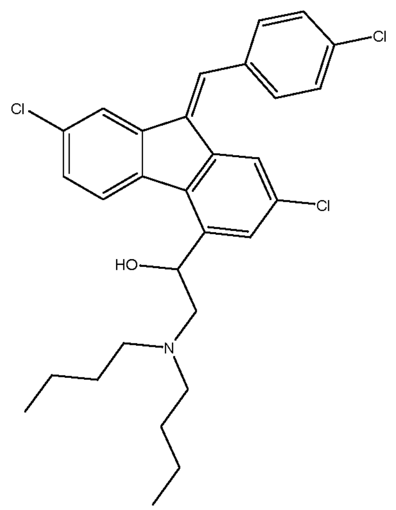

Formula I.

In the twelfth aspect of the present invention, the present invention provides use of a pharmaceutical composition in the manufacture of a medicament for inhibiting the replication or proliferation of a coronavirus (preferably SARS-CoV-2) in a cell (e.g., mammalian cell), wherein the pharmaceutical composition comprises the compound represented by Formula A, or a stereoisomer, a pharmaceutically acceptable salt, a solvate or a hydrate thereof, and the compound represented by Formula A is as described above.

In some embodiments, the pharmaceutical composition further comprises one or more selected from the group consisting of arteether, artemether, artemisone, dihydroartemisinin, artesunate, arteannuin B, artemisinic acid, and artemisinin. In some preferred embodiments, the pharmaceutical composition further comprises artemether, for example, it can be a compound artemether-benflumetol tablet.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the thirteenth aspect of the present invention, the present invention provides the compound represented by Formula A, or a stereoisomer, a pharmaceutically acceptable salt, a solvate or a hydrate thereof, for use in inhibiting the replication or proliferation of a coronavirus (preferably SARS-CoV-2) in a cell (e.g., mammalian cell);

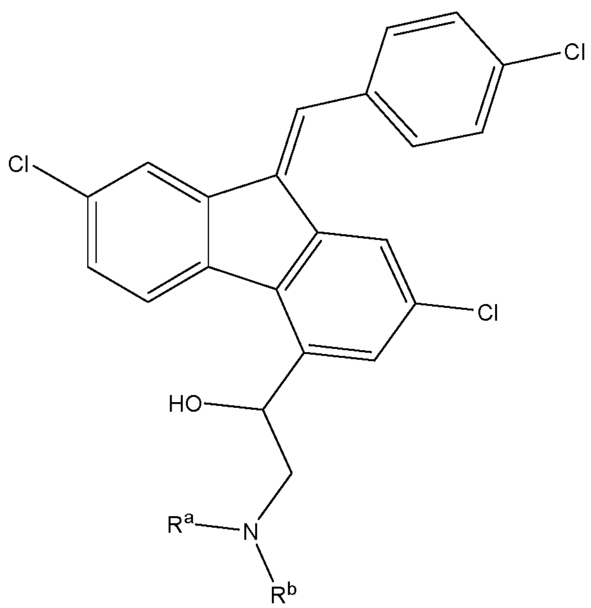

Formula A

wherein:
$R^a$ and $R^b$ are each independently selected from the group consisting of H, $C_1$-$C_6$ alkyl.

In some embodiments, $R^a$, $R^b$ are each independently selected from the group consisting of n-propyl, isopropyl, n-butyl, isobutyl, tert-butyl, sec-butyl.

In some embodiments, $R^a$, $R^b$ are each independently selected from the group consisting of n-butyl, isobutyl, tert-butyl, sec-butyl.

In some embodiments, the structure of the compound of Formula A is represented by Formula I,

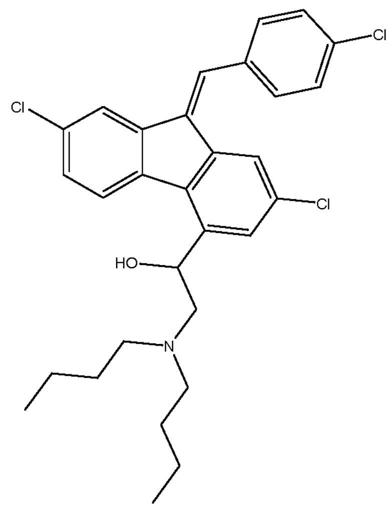

Formula I.

In the fourteenth aspect of the present invention, the present invention provides a pharmaceutical composition for use in inhibiting the replication or proliferation of a coronavirus (preferably SARS-CoV-2) in a cell (e.g., mammalian cell), wherein the pharmaceutical composition comprises the compound represented by Formula A, or a stereoisomer, a pharmaceutically acceptable salt, a solvate or a hydrate thereof, the compound represented by Formula A is as described above.

In some embodiments, the pharmaceutical composition further comprises one or more selected from the group consisting of arteether, artemether, artemisone, dihydroartemisinin, artesunate, arteannuin B, artemisinic acid, and artemisinin. In some preferred embodiments, the pharmaceutical composition further comprises artemether, for example, it can be a compound artemether-benflumetol tablet.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the fifteenth aspect of the present invention, the present invention provides a method for inhibiting the replication or proliferation of a coronavirus (preferably SARS-CoV-2) in a cell (e.g., mammalian cell), comprising administering to the cell (e.g., mammalian cell) an effective amount of the compound represented by Formula A, or a stereoisomer, a pharmaceutically acceptable salt, a solvate or a hydrate thereof;

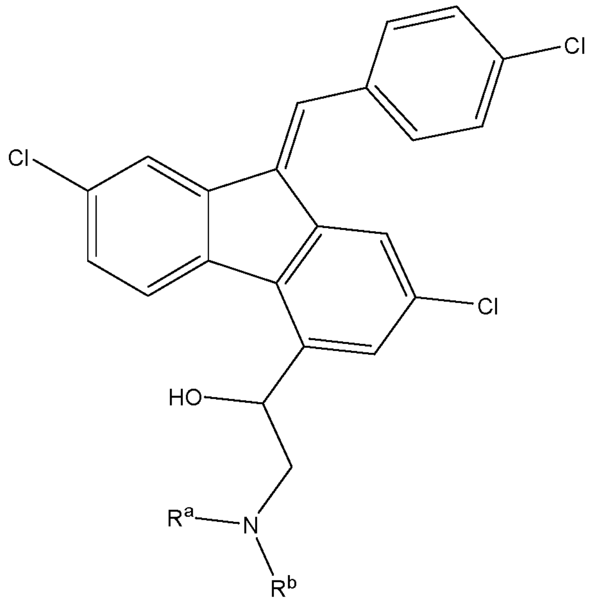

Formula A

wherein:
$R^a$ and $R^b$ are each independently selected from the group consisting of H, $C_1$-$C_6$ alkyl.

In some embodiments, $R^a$, $R^b$ are each independently selected from the group consisting of n-propyl, isopropyl, n-butyl, isobutyl, tert-butyl, sec-butyl.

In some embodiments, $R^a$, $R^b$ are each independently selected from the group consisting of n-butyl, isobutyl, tert-butyl, sec-butyl.

In some embodiments, the structure of the compound of Formula A is represented by Formula I,

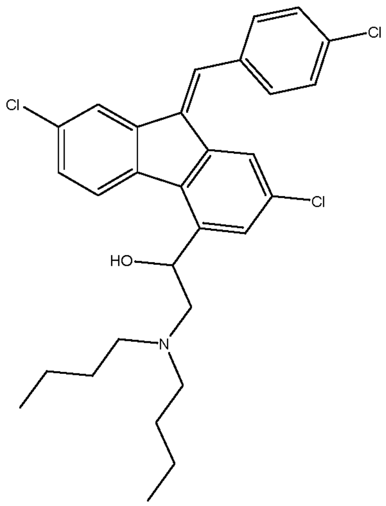

Formula I.

In the sixteenth aspect of the present invention, the present invention provides a method for inhibiting the replication or proliferation of a coronavirus (preferably SARS-CoV-2) in a cell (e.g., mammalian cell), comprising administering to the cell (e.g., mammalian cell) an effective amount of a pharmaceutical composition, wherein the pharmaceutical composition comprises the compound represented by Formula A, or a stereoisomer, a pharmaceutically acceptable salt, a solvate or a hydrate thereof, and the compound represented by Formula A is as described above.

In some embodiments, the pharmaceutical composition further comprises one or more selected from the group consisting of arteether, artemether, artemisone, dihydroartemisinin, artesunate, arteannuin B, artemisinic acid, and

artemisinin. In some preferred embodiments, the pharmaceutical composition further comprises artemether, for example, it can be a compound artemether-benflumetol tablet.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In some embodiments, the mammal includes bovine, equine, ovine, porcine, canine, feline, rodent, primate, and for example, is a human, cat, dog or pig.

In the present invention, the official classification name of the term "2019 novel coronavirus (2019-nCoV)" is severe acute respiratory syndrome coronavirus 2 (SARS-CoV-2).

In the present invention, the official name of the term "disease caused by 2019 novel coronavirus (2019-nCoV)" is COVID-19.

In the present invention, "subject" refers to a vertebrate. In certain embodiments, the vertebrate refers to a mammal. The mammal includes bovine, equine, ovine, porcine, canine, feline, rodent, primate, and for example is a human, cat, dog or pig. The mammal includes, but is not limited to, livestock (e.g., cattle), pet (e.g., cat, dog, and horse), primate, mouse, and rat. In certain embodiments, the mammal refers to a human.

In the present invention, the term "therapeutically effective amount" or "prophylactically effective amount" refers to an amount sufficient to treat or prevent a patient's disease but low enough to avoid serious side effects (at a reasonable benefit/risk ratio) within the scope of sound medical judgment. The therapeutically effective amount of a compound will depend on the particular compound selected (e.g., taking into account the potency, effectiveness and half-life of the compound), the route of administration selected, the disease being treated, the severity of the disease being treated, the age, size, weight and physical ailment of the patient being treated, the medical history of the patient being treated, the duration of treatment, the nature of concurrent therapy, the desired therapeutic effect, and the like, but can still be routinely determined by those skilled in the art.

In addition, it should be pointed out that the specific dosage and usage of the compound represented by the Formula A, or its stereoisomer, or its pharmaceutically acceptable salt and/or its solvate and/or its hydrate for different patients are determined by many factors, including the patient's age, weight, gender, natural health status, nutritional status, the active strength of drug, the time of administration, metabolic rate, the severity of condition, and the subjective judgment of the attending physician. It is preferred here to use a dose between 0.001 and 1000 mg/kg body weight/day.

The pharmaceutically acceptable salt of the compound represented by the Formula A of the present invention includes its inorganic or organic acid salt and its inorganic or organic base salt, and the present invention relates to all forms of the above-mentioned salts, including but not limited to: sodium salt, potassium salt, calcium salt, lithium salt, meglumine salt, hydrochloride, hydrobromide, hydroiodide, nitrate, sulfate, hydrosulfate, phosphate, hydrophosphate, acetate, propionate, butyrate, oxalate, trimethylacetate, adipate, alginate, lactate, citrate, tartrate, succinate, maleate, fumarate, picrate, aspartate, gluconate, benzoate, mesylate, ethanesulfonate, benzenesulfonate, p-toluenesulfonate and pamoate, etc.

The pharmaceutical compositions involved in the present invention may comprise a pharmaceutically acceptable carrier, including but not limited to: ion exchanger, alumina, aluminum stearate, lecithin, serum protein such as human serum albumin, buffer substance such as phosphate, glycerol, sorbic acid, potassium sorbate, partial glyceride mixture of saturated vegetable fatty acids, water, salt or electrolyte such as protamine sulfate, disodium hydrogen phosphate, potassium hydrogen phosphate, sodium chloride, zinc salt, colloidal silica, magnesium trisilicate, polyvinylpyrrolidone, cellulosic substance, polyethylene glycol, sodium carboxymethylcellulose, polyacrylate, beeswax, lanolin.

The pharmaceutical composition of the present invention can be prepared into various forms according to different administration routes.

According to the present invention, the pharmaceutical composition can be administered in any of the following ways: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or via an explanted reservoir. Of these, oral, intraperitoneal or intravenous administration is preferred.

For oral administration, the compound represented by Formula A, its stereoisomer or its pharmaceutically acceptable salt and/or its solvate and/or its hydrate can be made into any orally acceptable preparation, including but not limited to tablet, capsule, aqueous solution or aqueous suspension. Among them, the commonly used carrier for tablet includes lactose and corn starch, and lubricant such as magnesium stearate may also be added. Commonly used diluent for capsule preparation includes lactose and dried cornstarch. Aqueous suspension is usually prepared by mixing the active ingredient with suitable emulsifying agent and suspending agent. If desired, some sweetening, flavoring or coloring agents may also be added to the above oral preparations.

For rectal administration, the compound represented by Formula A, its stereoisomer or its pharmaceutically acceptable salt and/or its solvate and/or its hydrate can generally be made into the form of suppository, which is prepared by mixing the drug with a suitable non-irritating excipient. The excipient is solid at room temperature, but melts at rectal

temperature to release the drug. Such excipient includes cocoa butter, beeswax and polyethylene glycol.

For topical administration, especially when treating affected surfaces or organs easily accessible by topical administration, such as eye, skin or lower intestinal neurological diseases, the compound represented by Formula A, its stereoisomer or its pharmaceutically acceptable salt and/or its solvate and/or its hydrate can be made into different topical preparations according to different affected surfaces or organs, and the specific instructions are as follows:

For topical administration to the eye, the compound of Formula A, its stereoisomer or its pharmaceutically acceptable salt and/or its solvate and/or its hydrate can be formulated into the form of a micronized suspension or solution, the carrier used is isotonic sterile saline with a certain pH, which may or may not be added with a preservative such as benzyl alkoxide chloride. In addition, for ophthalmic use, the compound can be formulated into the form of an ointment such as petrolatum ointment.

For topical administration to the skin, the compound represented by Formula A, its stereoisomer or its pharmaceutically acceptable salt and/or its solvate and/or its hydrate can be made into the form of a suitable ointment, lotion or cream, in which the active ingredient is suspended or dissolved in one or more carriers. The carriers that can be used in ointment here include, but are not limited to: mineral oil, liquid petrolatum, white petrolatum, propylene glycol, polyethylene oxide, polypropylene oxide, emulsified wax and water; the carriers that can be used in lotion or cream include, but are not limited to: mineral oil, sorbitan monostearate, Tween 60, cetyl ester wax, cetenylaryl alcohol, 2-octyldodecanol, benzyl alcohol and water.

For topical administration to the lower intestinal tract, the compound represented by the Formula A, its stereoisomer or its pharmaceutically acceptable salt and/or its solvate and/or its hydrate can be made into the above-mentioned rectal suppository preparation or suitable form of enema preparation. In addition, topical transdermal patches can also be used.

The compound represented by the Formula A, its stereoisomer or its pharmaceutically acceptable salt and/or its solvate and/or its hydrate can also be administered in the form of sterile injectable preparation, including sterile injectable water or oil suspension, or sterile injectable solution. Among them, the carriers and solvents that can be used are water, Ringer's solution and isotonic sodium chloride solution. In addition, sterile nonvolatile oil such as monoglyceride or diglyceride can also be used as a solvent or suspending medium.

The medicaments in the above-mentioned various dosage forms can be prepared according to the conventional methods in the pharmaceutical field.

In various parts of this specification, the substituents of the compounds disclosed in the present invention are disclosed in terms of group type or range. Specifically, the present invention includes each and every independent subcombination of each member of these group types and ranges. For example, the term "$C_1$-$C_6$ alkyl" specifically refers to independently disclosed methyl, ethyl, C3 alkyl, C4 alkyl, C5 alkyl and C6 alkyl groups.

In addition, it should be noted that, unless it is clearly stated otherwise, the expression "each independently ..." used herein should be understood in a broad sense, which refers to that the specific options represented by different symbols do not affect each other, and can be the same or different.

The term "$C_1$-$C_6$ alkyl" refers to any straight or branched chain group containing 1 to 6 carbon atoms, such as methyl, ethyl, n-propyl, isopropyl, n-butyl, isobutyl, tert-butyl, sec-butyl, n-pentyl, tert-amyl, n-hexyl, etc.

## Brief Description of the Drawings

Figure 1 shows that benflumetol effectively reduced viral nucleic acid load on vero E6 cells infected with SARS-CoV-2. Benflumetol was able to inhibit the viral RNA load on cells 48h after the cells were infected with SARS-CoV-2, and the inhibitory activity was dose-dependent. The left ordinate is the percentage inhibition rate calculated according to the copy number of viral RNA in the sample (corresponding to the dots and their fitting lines in the figure), and the right ordinate is the percentage toxicity calculated according to the cell viability (corresponding to the squares and their fitting lines in the figure), and the abscissa is the concentration of the drug (benflumetol, i.e., lumefantrine).

## Specific Models for Carrying Out the Invention

The examples of the present invention will be described in detail below. The examples described below with reference to the accompanying drawings are exemplary, and are intended to explain the present invention, but should not be construed as a limitation of the present invention.

The present invention provides a compound having the structure of Formula I, its stereoisomer, its pharmaceutically acceptable salt and/or its solvate and/or its hydrate:

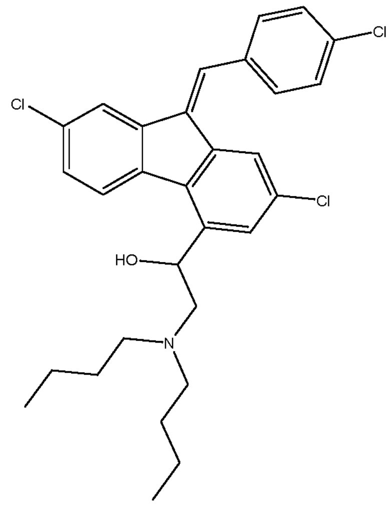

Formula I

In certain embodiments, the pharmaceutically acceptable salt of the compound of Formula I of the present invention includes its inorganic or organic acid salts, and inorganic or organic base salts, and the present invention relates to all forms of the above-mentioned salts, including but not limited to: sodium salt, potassium salt, calcium salt, lithium salt, meglumine salt, hydrochloride, hydrobromide, hydroiodide, nitrate, sulfate, hydrosulfate, phosphate, hydrophosphate, acetate, propionate, butyrate, oxalate, trimethylacetate, adipate, alginate, lactate, citrate, tartrate, succinate, maleate, fumarate, picrate, aspartate, gluconate, benzoate, mesylate, ethanesulfonate, benzenesulfonate, p-toluenesulfonate and pamoate, etc.

The compound of Formula I can inhibit viral replication on cells and reduce viral nucleic acid load in a cell culture.

The present invention relates to use of the compound represented by Formula I, its stereoisomer or its pharmaceutically acceptable salt and/or its solvate and/or its hydrate in the manufacture of a medicament for the treatment of a disease or infection caused by a coronavirus, especially SARS-CoV-2 (including but not limited to respiratory disease (e.g., simple infection such as fever, cough and sore throat, etc., pneumonia, acute or severe acute respiratory infection, hypoxic respiratory failure and acute respiratory distress syndrome, sepsis and septic shock, severe acute respiratory syndrome, etc.)),

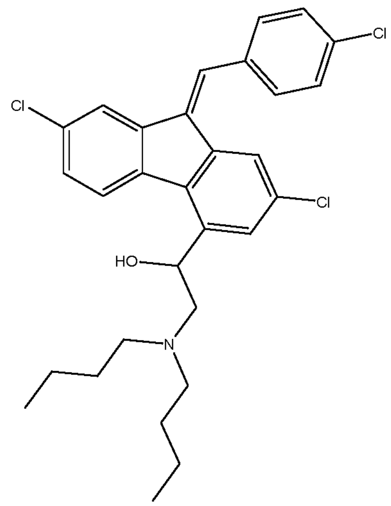

Formula I

The present invention also relates to use of the compound represented by Formula I, its stereoisomer, its pharmaceutically acceptable salt and/or its solvate and/or its hydrate in the manufacture of a medicament as a coronavirus inhibitor.

The present invention also relates to use of the compound represented by Formula I, its stereoisomer, its pharmaceutically acceptable salt and/or its solvate and/or its hydrate in the manufacture of a medicament for inhibiting the replication or proliferation of a coronavirus in a cell (e.g., mammalian cell).

The present invention also relates to a pharmaceutical composition, which comprises the compound represented by the Formula I, its stereoisomer or its pharmaceutically acceptable salt and/or its solvate and/or its hydrate, preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to use of the pharmaceutical composition comprising the compound represented by the Formula I, its stereoisomer or its pharmaceutically acceptable salt and/or its solvate and/or its hydrate, or the compound represented by the Formula I, its stereoisomer or its pharmaceutically acceptable salt and/or its solvate and/or its hydrate, in the manufacture of a medicament for the treatment of a disease including respiratory disease but not limited to respiratory disease (including simple infection such as fever, cough and sore throat, etc., pneumonia, acute or severe acute respiratory infection, hypoxic respiratory failure and acute respiratory distress syndrome, sepsis and septic shock, severe acute respiratory syndrome, etc.).

The present invention also relates to a method for treating and/or preventing a disease in a mammal in need thereof or a method for inhibiting the replication or proliferation of a coronavirus in a mammal in need thereof, the method comprises administering to the mammal in need a therapeutically and/or prophylactically effective amount of the pharmaceutical composition comprising the compound represented by the Formula I, its stereoisomer or its pharmaceutically acceptable salt and/or its solvate and/or its hydrate, or the compound represented by the Formula I, its stereoisomer or its pharmaceutically acceptable salt and/or its solvate and/or its hydrate, wherein the disease includes a disease caused by a coronavirus.

In certain embodiments, the disease caused by a coronavirus, especially SARS-CoV-2, includes, but is not limited to, respiratory disease (e.g., simple infection such as fever, cough and sore throat, etc., pneumonia, acute or severe acute respiratory infection, hypoxic respiratory failure and acute respiratory distress syndrome, sepsis and septic shock, severe acute respiratory syndrome, etc.).

The present invention also relates to use of a pharmaceutical composition in the manufacture of a medicament for the treatment of a disease or infection caused by a coronavirus, especially SARS-CoV-2 (e.g., respiratory disease (e.g., simple infection such as fever, cough and sore throat, etc., pneumonia, acute or severe acute respiratory infection, hypoxic respiratory failure and acute respiratory distress syndrome, sepsis and septic shock, severe acute respiratory syndrome, etc.), wherein the pharmaceutical composition comprises the compound represented by Formula I, its stereoisomer or its pharmaceutically acceptable salt and/or its solvate and/or its hydrate,

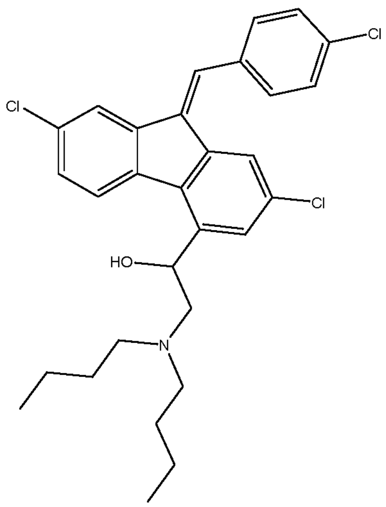

Formula I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to use of a pharmaceutical composition in the manufacture of a medicament as a coronavirus inhibitor, wherein the pharmaceutical composition comprises the compound represented by Formula

I, its stereoisomer, its pharmaceutically acceptable salt and/or its solvate and/or its hydrate,

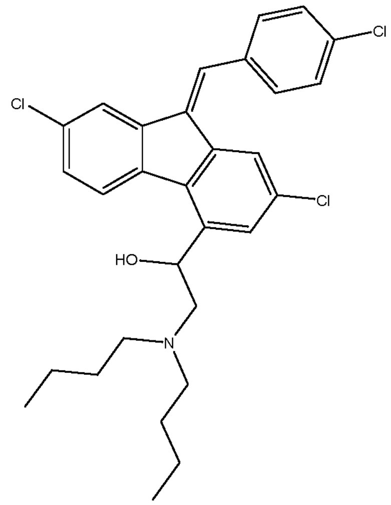

Formula I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to use of a pharmaceutical composition in the manufacture of a medicament for inhibiting the replication or proliferation of a coronavirus in a cell (e.g., mammalian cell), wherein the pharmaceutical composition comprises the compound represented by Formula I, its stereoisomer, its pharmaceutically acceptable salt and/or its solvate and/or its hydrate,

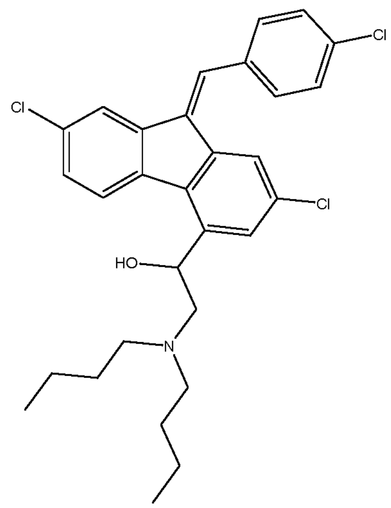

Formula I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to the compound represented by Formula I, its stereoisomer or its pharmaceutically acceptable salt and/or its solvate and/or its hydrate, for use in the treatment of a disease or infection caused by a coronavirus, especially SARS-CoV-2 (including but not limited to respiratory disease (e.g., simple infection such as fever, cough and sore throat, etc., pneumonia, acute or severe acute respiratory infection, hypoxic respiratory failure and acute respiratory distress syndrome, sepsis and septic shock, severe acute respiratory syndrome, etc.)).

The present invention also relates to the compound represented by Formula I, its stereoisomer, its pharmaceutically acceptable salt and/or its solvate and/or its hydrate, which is used as a coronavirus inhibitor.

The present invention also relates to the compound represented by Formula I, its stereoisomer, its pharmaceutically acceptable salt and/or its solvate and/or its hydrate, for use in inhibiting the replication or proliferation of a coronavirus in a cell (e.g., mammalian cell).

The present invention also relates to a pharmaceutical composition for use in the treatment of a disease or infection caused by a coronavirus, especially SARS-CoV-2 (e.g., respiratory disease (e.g., simple infection such as fever, cough and sore throat, etc., pneumonia, acute or severe acute respiratory infection, hypoxic respiratory failure and acute respiratory distress syndrome, sepsis and septic shock, severe acute respiratory syndrome, etc.) ), wherein the pharmaceutical composition comprises the compound represented by the Formula I, its stereoisomer or its pharmaceutically acceptable salt and/or its solvate and/or its hydrate,

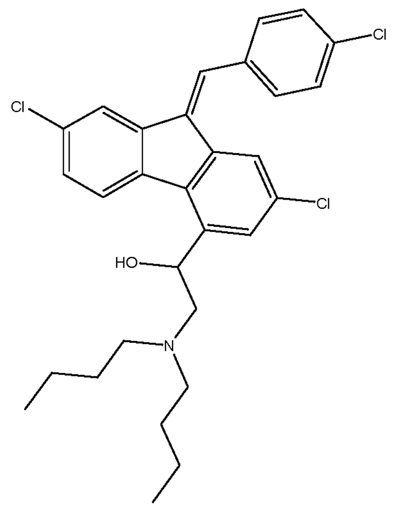

Formula I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to a pharmaceutical composition, which is used as a coronavirus inhibitor, wherein the pharmaceutical composition comprises the compound represented by Formula I, its stereoisomer, its pharmaceutically acceptable salt and/or its solvate and/ or its hydrate,

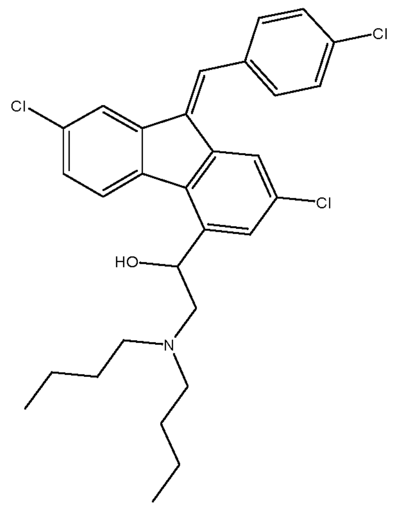

Formula I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to a pharmaceutical composition, for use in inhibiting the replication or proliferation of a coronavirus in a cell (e.g., mammalian cell), wherein the pharmaceutical composition comprises the compound represented by Formula I, its stereoisomer, its pharmaceutically acceptable salt and/or its solvate and/or its hydrate,

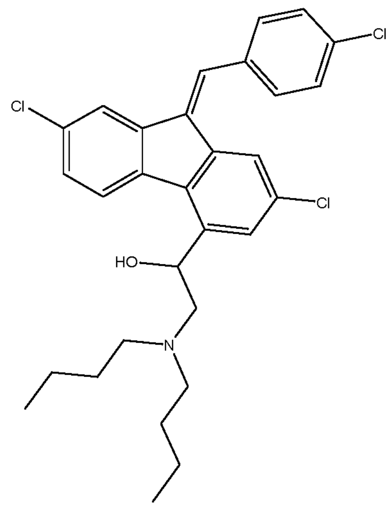

Formula I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

In certain embodiments, the coronavirus of the present invention is SARS-CoV-2.

In certain embodiments, the disease caused by the coronavirus of the present invention is the disease caused by SARS-CoV-2, namely COVID-19.

In certain embodiments, the mammal of the present invention includes bovine, equine, ovine, porcine, canine, feline, rodent, primate, for example is a human, cat, dog or pig.

The present invention will be further explained below in conjunction with specific examples.

Example 1: Experiment of reduction of viral nucleic acid load of SARS-CoV-2-infected cells by benflumetol (i.e., the compound represented by Formula I)

(1) Drug treatment of virus-infected cells

Vero E6 cells (purchased from ATCC, Cat. No. 1586) were inoculated into a 24-well plate and cultured for 24 hours; then virus infection was performed, specifically, SARS-CoV-2 (2019-nCoV) virus (nCoV-2019BetaCoV/Wuhan/WIV04/2019 strain, provided by Wuhan Institute of Virology, Chinese Academy of Sciences) was diluted to the corresponding concentration with 2% cell maintenance solution (formulation was: FBS (purchased from Gibco, Cat. No. 16000044) was added to MEM (purchased from Gibco, Cat. No. 10370021) at a volume ratio of 2% to form 2% cell maintenance solution), and then added to the 24-well plate so that each well had a virus amount of 100 $TCID_{50}$. Next, benflumetol (purchased from Selleck, Cat. No. S3746) was diluted to corresponding concentrations with 2% cell maintenance solution and added to the corresponding wells, so that the final drug concentrations were 100 μM, 50 μM, 25 μM, 12.5 μM, 6.25μM, and 3.13μM, respectively, and then incubation was performed in a 37°C, 5% $CO_2$ incubator for 48h; while 2% cell maintenance solution without any test drug was added only in the cell control group.

(2) RNA extraction

RNA extraction kit was purchased from Qiagen, Cat. No. 74106. The consumables (spin column, RNase-free 2mL collection tubes, etc.) and reagents (RLT, RW1, RPE, RNase-free water, etc.) involved in the following RNA extraction steps were all parts of the kit. The following extraction steps were all those recommended by the kit instructions.

1) 100 μL of the supernatant of the test culture plate was taken, added to a nuclease-free EP tube, then added with 350 μL of Buffer RLT, blown and mixed with a pipette gun for fully lysing, and then centrifuged to get a supernatant;

2) an equal volume of 70% ethanol was added to the supernatant obtained in 1), and mixed well;

3) the mixture obtained in the above 2) was transferred into an RNase-free spin column, centrifuged at 12,000 rpm for 15s, and the waste liquid was discarded;

4) 700 μL of Buffer RW1 was added to the spin column, centrifuged at 12000 rpm for 15s to wash the spin column, and the waste liquid was discarded;

5) 500 μL of Buffer RPE was added to the spin column, centrifuged at 12000 rpm for 15s to wash the spin column, and the waste liquid was discarded;

6) 500 μL of Buffer RPE was added to the spin column, centrifuged at 12000 rpm for 2 min to wash the spin column, and the waste liquid was discarded;

7) a new 2mL RNase-free collection tube was used for placement of the spin column, centrifugation was performed at 12,000 rpm for 1 min, the spin column was dried, and then the entire spin column was transferred to the 1.5mL collection tube of step 8);

8) the spin column dried in step 7) was placed in a new 1.5mL collection tube, 30μL of RNase-free water was added to the spin column, centrifuged at 12,000 rpm for 2 minutes, and the eluate contained the corresponding RNA, RNase inhibitor (purchased from NEB company, Cat. No. M0314L) was added, and each RNA concentration was detected with Nano Drop (purchased from Thermo scientific, model Nano Drop One).

(3) RNA reverse transcription

The reverse transcription kit (PrimeScript™ RT reagent Kit with gDNA Eraser, Cat. No. RR047Q) produced by TaKaRa Company was used for RNA reverse transcription. The steps were as follows.

① gDNA removal: RNA samples were collected from each experimental group, and 1 μg thereof was taken for reverse transcription. First, 2 μL of 5× gDNA Eraser Buffer was added to the RNA of each experimental group, RNase-free water was used to make up the reaction system to 10 μL, then the reaction system was mixed well, and placed in a water bath at 42°C for 2 min to remove gDNA that could exist in the sample;

② reverse transcription: enzyme, primer Mix and reaction buffer at appropriate amounts were added to the sample obtained in ①, RNase Free water was used to make up the volume to 20 μL, reaction was performed in a water bath at 37°C for 15 minutes, and then performed in a water bath at 85°C for 5 sec, to obtain cDNA via transcription.

(4) Real-time PCR

Fluorescence quantitative PCR was used to detect the copy number per milliliter of the original virus solution.

The reaction system was mixed using TB Green Premix (Takara, Cat#RR820A), and the amplification reaction and reading were performed by a StepOne Plus Real-time PCR machine (brand: ABI). The number of copies per milliliter of the original virus solution was calculated. The steps were as follows:

① establishment of standard product: plasmid pMT-RBD (plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL, respectively. 2 μL of standard or cDNA template was taken for qPCR reaction.
② the primer sequences used in the experiment were as follows (all were indicated in the 5'-3' direction):

RBD-qF: CAATGGTTTAACAGGCACAGG (SEQ ID NO: 1)
RBD-qR: CTCAAGTGTCTGTGGATCACG (SEQ ID NO: 2)

③ the reaction procedure was as follows:

pre-denaturation: 95°C for 5 minutes;
cycling parameters: 95°C for 15 seconds, 54°C for 15 seconds, and 72°C for 30 seconds, a total of 40 cycles.

Inhibition rate (%) = (number of RNA copies in drug treatment group) / (number of RNA copies in cell infection group) × 100%

(5) Detection of drug cytotoxicity

The detection of drug cytotoxicity was performed by CCK-8 kit (Beoytime). Specific steps were as follows:

① $1\times10^4$ Vero E6 (ATCC) cells were inoculated in a 96-well plate and cultured at 37°C for 8 hours.
② The drug was diluted with DMSO to obtain a stock solution with an appropriate concentration, and then diluted with MEM medium (purchased from Gibco, Cat. No. 10370021) containing 2% FBS (purchased from Gibco, Cat. No. 16000044) to the same concentration as that for the drug treatment. The original medium in the 96-well plate was discarded, 100 μL of drug-containing MEM medium was added to the cells, and three replicate wells were set for each concentration. Negative control (adding DMSO and medium to the cell wells, without adding drug) and blank control (no cells, adding DMSO and medium) were set. After the drug was added, the cells were cultured at 37°C for 48 hours.
③ 20 μL of CCK-8 solution (Beoytime) was added to the wells to be tested, mixed gently without generating air bubbles, and incubation was continued at 37°C for 2 hours. $OD_{450}$ was read on a microplate reader (purchased from Molecular Devices, model: SpectraMax M5), and cell viability was calculated:

$$\text{Cell viability (\%)} = (A_{\text{(drug treatment group)}} - A_{\text{(blank control)}}) / (A_{\text{(negative control)}} - A_{\text{(blank control)}}) \times 100\%$$

wherein A represents reading of microplate reader.

(6) Experimental results

The results of the virus proliferation inhibition experiment showed that the test compound could effectively inhibit the replication of the SARS-CoV-2 virus genome in the infection supernatant at the concentrations of 100 μM, 50 μM and 25 μM. (Table 1 and Figure 1)

Table 1. Results of antiviral experiments of benflumetol

| Concentration (μM) | 100 | 50 | 25 | 12.5 | 6.25 | 3.13 | Vehicle |
|---|---|---|---|---|---|---|---|
| Viral genome copy number (MOI = 0.05) | 2927366 ± 40605 | 20512679 ± 4610588 | 48801530 ± 4509346 | 87790130 ± 9400381 | 87462582 ± 2932239 | 125717580 ± 9978188 | 108280612 ± 21937000 |

The cytotoxicity results showed that the treatment of the test compound (benflumetol) did not change the cell viability at all test concentrations, that was, the test compound had no toxic effect on the cells at all concentrations (Table 2 and Figure 1).

Table 2. Cytotoxicity test of benflumetol

| Concentration (μM) | 100 | 50 | 25 | 12.5 | 6.25 | 3.13 | 1.56 | 0.78 | Vehicle |
|---|---|---|---|---|---|---|---|---|---|
| Cell viability (% of negative control) | 122.12 + 1.43 | 115.52 + 5.83 | 113.54 + 2.77 | 108.69 + 0.56 | 106.70+ 10.62 | 96.69 + 3.31 | 94.55 + 4.86 | 92.88 + 6.79 | 100 + 3.22 |

The specific embodiments described above further describe the purpose, technical solutions and beneficial effects of the present invention in detail. It should be understood that the above-mentioned specific embodiments are only specific embodiments of the present invention, and are not intended to limit the scope of the present invention. Any modification, equivalent replacement, improvement, etc. made within the spirit and principle of the present invention shall be included in the protection scope of the present invention.

## Claims

**1.** Use of a compound represented by Formula A, or a stereoisomer, a pharmaceutically acceptable salt, a solvate or a hydrate thereof in the manufacture of a medicament for the treatment of a disease or infection caused by a coronavirus (preferably SARS-CoV-2);

preferably, the disease caused by SARS-CoV-2 is COVID-19;
preferably, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.;
preferably, the simple infection includes, but is not limited to, fever, cough, sore throat, nasal congestion, fatigue, headache, muscle pain or discomfort; preferably, the mild pneumonia includes, but is not limited to, cough, dyspnea and/or tachypnea; preferably, the severe pneumonia includes, but is not limited to, increased respiratory rate, severe respiratory failure or dyspnea, central cyanosis, lethargy, unconsciousness or convulsions, gasp; preferably, the acute respiratory distress syndrome includes, but is not limited to, pulmonary edema; preferably, the sepsis includes, but is not limited to, organ dysfunction;

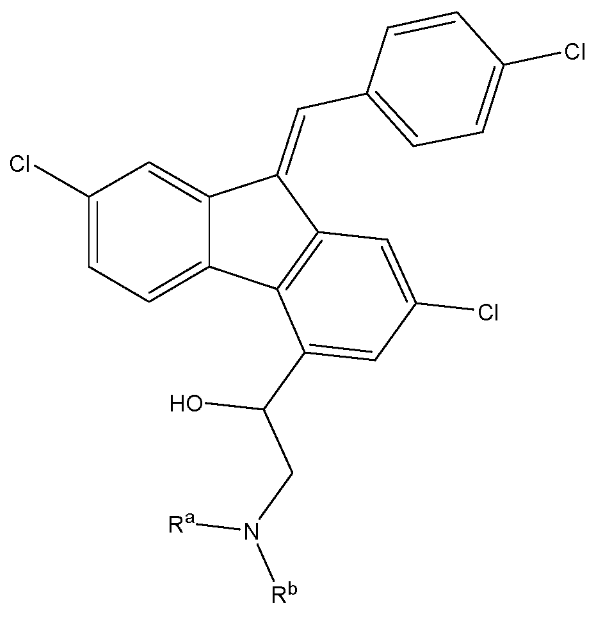

Formula A

wherein:

$R^a$ and $R^b$ are each independently selected from the group consisting of H, $C_1$-$C_6$ alkyl;

preferably, $R^a$ and $R^b$ are each independently selected from the group consisting of n-propyl, isopropyl, n-butyl, isobutyl, tert-butyl, and sec-butyl;
more preferably, $R^a$ and $R^b$ are each independently selected from the group consisting of n-butyl, isobutyl, tert-butyl, and sec-butyl.

**2.** The use according to claim 1, wherein the structure of the compound represented by Formula A is represented by Formula I,

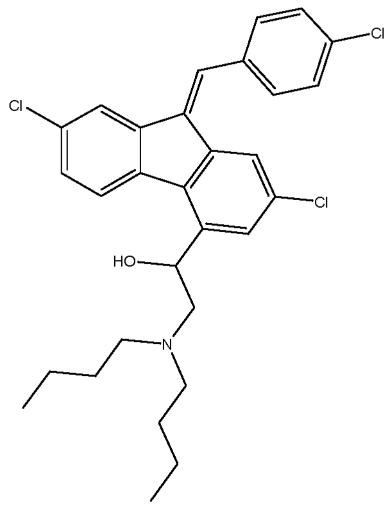

Formula I.

**3.** Use of a pharmaceutical composition in the manufacture of a medicament for treating a disease or infection caused by a coronavirus (preferably SARS-CoV-2), wherein the pharmaceutical composition comprises the compound represented by Formula A, or a stereoisomer, a pharmaceutically acceptable salt, a solvate or a hydrate thereof, and the compound represented by Formula A is as defined in any one of claims 1 to 2;

preferably, the disease caused by SARS-CoV-2 is COVID-19;
preferably, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.;
preferably, the simple infection includes, but is not limited to, fever, cough, sore throat, nasal congestion, fatigue, headache, muscle pain or discomfort; preferably, the mild pneumonia includes, but is not limited to, cough, dyspnea and/or tachypnea; preferably, the severe pneumonia includes, but is not limited to, increased respiratory rate, severe respiratory failure or dyspnea, central cyanosis, lethargy, unconsciousness or convulsions, gasp; preferably, the acute respiratory distress syndrome includes, but is not limited to, pulmonary edema; preferably, the sepsis includes, but is not limited to, organ dysfunction;
preferably, the pharmaceutical composition further comprises one or more selected from the group consisting of arteether, artemether, artemisone, dihydroartemisinin, artesunate, arteannuin B, artemisinic acid, and artemisinin; preferably, the pharmaceutical composition further comprises artemether;
preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

**4.** Use of a compound represented by Formula A, or a stereoisomer, a pharmaceutically acceptable salt, a solvate or a hydrate thereof in the manufacture of a medicament as a coronavirus (preferably SARS-CoV-2) inhibitor,

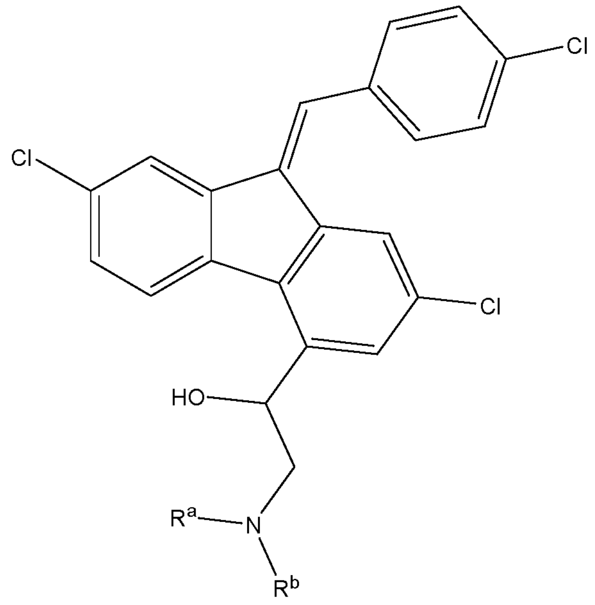

Formula A

wherein:

$R^a$ and $R^b$ are each independently selected from the group consisting of H, $C_1$-$C_6$ alkyl;
preferably, $R^a$ and $R^b$ are each independently selected from the group consisting of n-propyl, isopropyl, n-butyl, isobutyl, tert-butyl, and sec-butyl;
more preferably, $R^a$ and $R^b$ are each independently selected from the group consisting of n-butyl, isobutyl, tert-butyl, and sec-butyl.

**5.** The use according to claim 4, wherein the structure of the compound represented by Formula A is represented by Formula I,

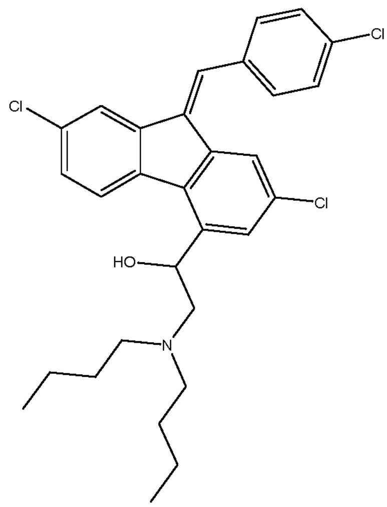

Formula I.

**6.** Use of a pharmaceutical composition in the manufacture of a medicament as a coronavirus (preferably SARS-CoV-2) inhibitor, wherein the pharmaceutical composition comprises a compound represented by Formula A, or a stereoisomer, a pharmaceutically acceptable salt, a solvate or a hydrate thereof, and the compound represented by Formula A is as defined in any one of claims 4 to 5;

preferably, the pharmaceutical composition further comprises one or more selected from the group consisting of arteether, artemether, artemisone, dihydroartemisinin, artesunate, arteannuin B, artemisinic acid, and artemisinin; preferably, the pharmaceutical composition further comprises artemether;
preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

**7.** Use of a compound represented by Formula A, or a stereoisomer, a pharmaceutically acceptable salt, a solvate or a hydrate thereof in the manufacture of a medicament for inhibiting the replication or proliferation of a coronavirus (preferably SARS-CoV-2) in a cell (e.g., mammalian cell);

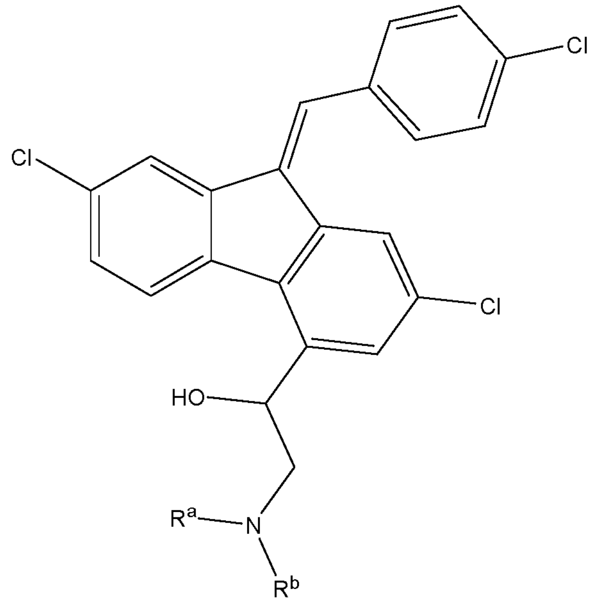

Formula A

wherein:

$R^a$ and $R^b$ are each independently selected from the group consisting of H, $C_1$-$C_6$ alkyl;
preferably, $R^a$ and $R^b$ are each independently selected from the group consisting of n-propyl, isopropyl, n-butyl, isobutyl, tert-butyl, and sec-butyl;
more preferably, $R^a$ and $R^b$ are each independently selected from the group consisting of n-butyl, isobutyl, tert-butyl, and sec-butyl.

**8.** The use according to claim 7, wherein the structure of the compound represented by Formula A is represented by Formula I,

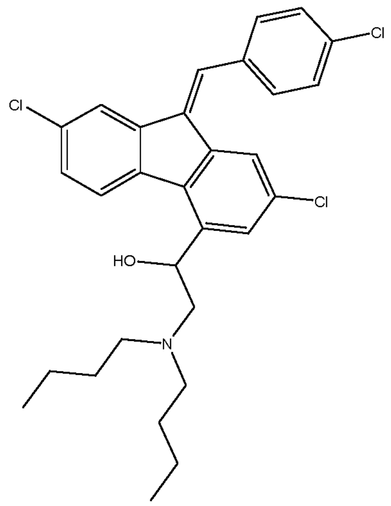

Formula I.

**9.** Use of a pharmaceutical composition in the manufacture of a medicament for inhibiting the replication or proliferation of a coronavirus (preferably SARS-CoV-2) in a cell (e.g., mammalian cell), wherein the pharmaceutical composition comprises a compound represented by Formula A, or a stereoisomer, a pharmaceutically acceptable salt, a solvate or a hydrate thereof, and the compound represented by Formula A is as defined in any one of claims 7 to 8;

preferably, the pharmaceutical composition further comprises one or more selected from the group consisting of arteether, artemether, artemisone, dihydroartemisinin, artesunate, arteannuin B, artemisinic acid, and artemisinin; preferably, the pharmaceutical composition further comprises artemether;
preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

**10.** The use according to claim 7 or 9, wherein the mammal comprises bovine, equine, ovine, porcine, canine, feline, rodent, primate, such as a human, cat, dog or pig.

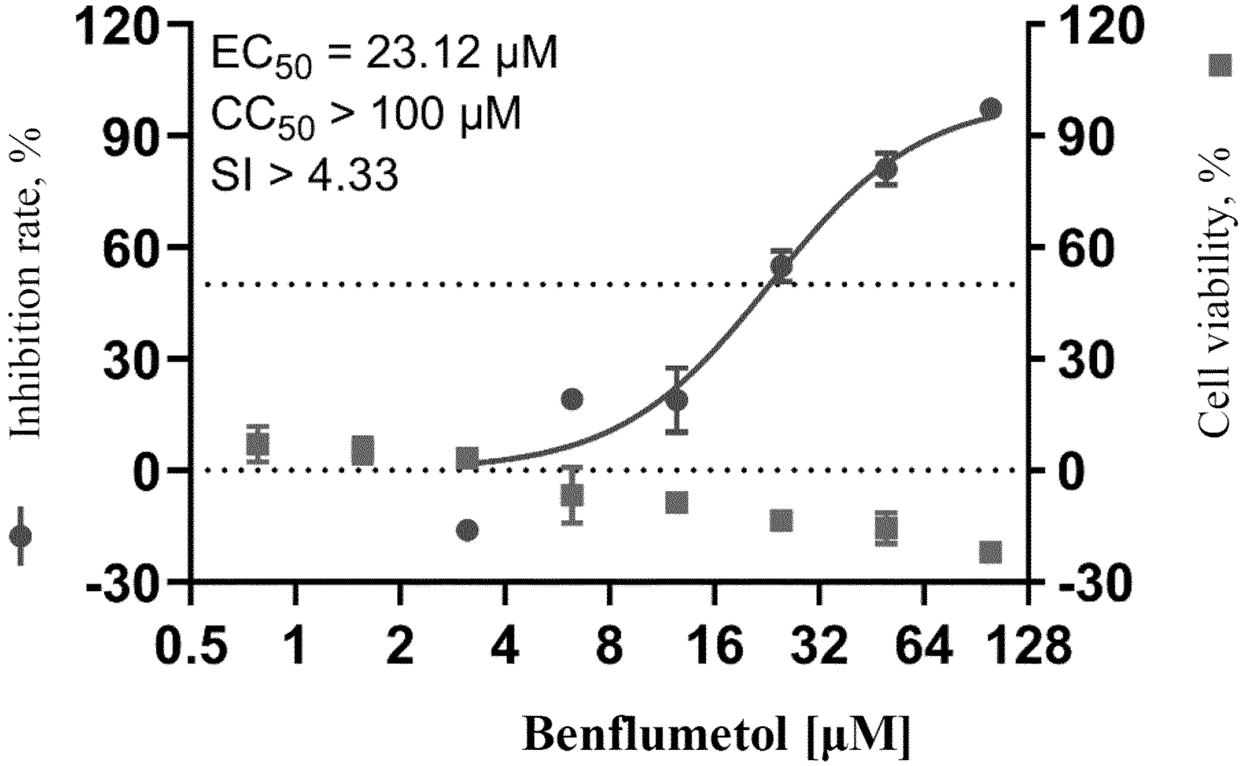
EC50 = 23.12 μM
CC50 > 100 μM
SI > 4.33
Inhibition rate, %
Cell viability, %
120
90
60
30
0
-30
0.5
1
2
4
8
16
32
64
128
Benflumetol [μM]

Figure 1

**INTERNATIONAL SEARCH REPORT**

International application No.
**PCT/CN2021/107739**

**A. CLASSIFICATION OF SUBJECT MATTER**

A61K 31/137(2006.01)i; A61K 45/06(2006.01)i; A61P 31/14(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, USTXT, EPTXT, STNext, CNKI, 百度学术, ISI_Web of Science, PUBMED, 军事科学院, 军事医学研究院, 曹瑞源, 王曼丽, 李薇, 赵磊, 杨晶晶, 李月香, 樊士勇, 周辛波, 肖典, 胡志红, 李松, 钟武, 苯芴醇, 本芴醇, 苯勿醇, 冠状病毒, 新冠, 肺炎, based on structure search, benflumetol, lumefantrine, coronavirus, SARS, SARS-COV-2, COVID-19, SARI, 2019-ncov, 82186-77-4

**C. DOCUMENTS CONSIDERED TO BE RELEVANT**

| Category* | Citation of document, with indication, where appropriate, of the relevant passages | Relevant to claim No. |
|---|---|---|
| PX | CN 111686096 A (ACAD OF MILITARY SCIENCES PLA CHINA ACAD OF MILITARY MEDICAL SCIENCES) 22 September 2020 (2020-09-22)<br>claims 1-10 | 1-10 |
| PX | CN 111803491 A (ACAD OF MILITARY SCIENCES PLA CHINA ACAD OF MILITARY MEDICAL SCIENCES) 23 October 2020 (2020-10-23)<br>claims 3, 6, 9-10 | 3, 6, 9-10 |
| PX | CAO, Ruiyuan et al.,. "Anti-SARS-CoV-2 Potential of Artemisinins In Vitro," *ACS Infectious Diseases,* Vol. 6, 31 July 2020 (2020-07-31),<br>pp. 2524-2531 | 1-10 |
| PX | GENDROT, Mathieu et al.,. "Antimalarial artemisinin-based combination therapies (ACT) and COVID-19 in Africa: In vitro inhibition of SARS-CoV-2 replication by mefloquine-artesunate," *International Journal of Infectious Diseases,* Vol. 99, 31 October 2020 (2020-10-31),<br>pp. 437-440 | 3, 6, 9-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 September 2021** | **21 October 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/107739** |

**C. DOCUMENTS CONSIDERED TO BE RELEVANT**

| Category* | Citation of document, with indication, where appropriate, of the relevant passages | Relevant to claim No. |
|---|---|---|
| A | PEELE, K. Abraham et al.,. "Molecular docking and dynamic simulations for antiviral compounds against SARS-CoV-2: A computational study," *Informatics in Medicine Unlocked,* Vol. 19, 11 May 2020 (2020-05-11), 100345 pages 1-6 | 1-10 |
| A | UZUN, Tuğçenur et al. "Artesunate: could be an alternative drug to chloroquine in COVID-19 treatment?," *Uzun and Toptas Chin Med,* Vol. 15, No. 54, 28 May 2020 (2020-05-28), pp. 1-4 | 1-10 |
| A | 刘桂梅 等 (LIU, Guimei et al.). "青蒿素及其衍生物用于治疗新型冠状病毒肺炎的探讨 (Discussion on Artemisinin and Its Derivatives for Treatment of COVID-19)" *药物评价研究 (Drug Evaluation Research),* Vol. 43, No. 4, 30 April 2020 (2020-04-30), pp. 606-612 | 1-10 |
| A | SEHAILIA, Moussa et al.,. "Antimalarial-agent artemisinin and derivatives portray more potent binding to Lys353 and Lys31-binding hotspots of SARS-CoV-2 spike protein than hydroxychloroquine: potential repurposing of artenimol for COVID-19," *JOURNAL OF BIOMOLECULAR STRUCTURE AND DYNAMICS,* Vol. 39, No. 16, 22 July 2020 (2020-07-22), pp. 6184-6194 | 1-10 |

Form PCT/ISA/210 (second sheet) (January 2015)

**INTERNATIONAL SEARCH REPORT**

International application No.
**PCT/CN2021/107739**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2021/107739**

| Patent document cited in search report | Publication date (day/month/year) | Patent family member(s) | Publication date (day/month/year) |
|---|---|---|---|
| CN 111686096 A | 22 September 2020 | CN 111686096 B | 14 May 2021 |
| CN 111803491 A | 23 October 2020 | None | |

Form PCT/ISA/210 (patent family annex) (January 2015)